# EUROPEAN PATENT SPECIFICATION

(11) **EP 3 496 719 B1**
(45) Date of publication and mention of the grant of the patent: **14.06.2023**
(21) Application number: 17838880.7
(22) Date of filing: 28.07.2017
(51) Int. Cl.: A61K 31/5025, A61K 45/06, A61P 31/18, A61K 9/00, A61K 9/20, A61K 31/34, A61K 31/5365, A61K 31/5377

(54) **A MULTI-CLASS ANTI-RETROVIRAL COMPOSITION**
ANTIRETROVIRALE ZUSAMMENSETZUNG MIT MEHREREN KLASSEN
COMPOSITION ANTIRÉTROVIRALE MULTI-CLASSE

(30) Priority: 08.08.2016 IN 201641026996
(43) Date of publication of application: 19.06.2019
(73) Proprietor: Hetero Labs Limited, Hyderabad Telangana 500055 (IN)
(72) Inventor: BANDI, Parthasarathi Reddy, Hyderabad Telangana 500055 (IN); PODILE, Khadgapathi, Hyderabad Telangana 500055 (IN); TIWARI, Sunil, Deviprasad, Hyderabad Telangana 500055 (IN); NELLURI, Ramarao, Hyderabad Telangana 500055 (IN); VAMSI KIRAN, Atluri, Hyderabad Telangana 500055 (IN)
(74) Representative: ZBM Patents - Zea, Barlocci & Markvardsen
(86) International application number: PCT/IB2017/054592
(87) International publication number: WO 2018/029565

(56) References cited:
- WO-A1-2014/184553
- WO-A1-2016/016279
- US-A1- 2007 071 822
- US-A1- 2014 142 070
- US-A1- 2016 008 374
- US-A1- 2016 038 502
- US-A1- 2016 067 255
- EMILIE R ELLIOT ET AL: "Pharmacokinetics of dolutegravir with and without darunavir/cobicistat in healthy volunteers", JOURNAL OF ANTIMICROBIAL CHEMOTHERAPY., vol. 74, no. 1, 1 October 2018 (2018-10-01), pages 149-156, XP055671693, GB ISSN: 0305-7453, DOI: 10.1093/jac/dky384
- MARZOLINI, C et al.: "Cobicistat versus ritonavir boosting and differences in the drug-drug interaction profiles with co-medications", Journal of Antimicrobial Chemotherapy, vol. 71, March 2016 (2016-03), pages 1755-1758, XP055413065, DOI: doi:10.1093/jac/dkw032

## Description

### PRIORITY

This patent application claims priority to Indian patent application number IN 201641026996, filed on August 08, 2016.

### FIELD OF THE INVENTION

The present invention relates to an anti-retroviral composition. In particular, the present invention relates to a tablet composition comprising combination of multi-class drugs particularly darunavir, dolutegravir and cobicistat and process for preparing the same.

### BACKGROUND OF THE INVENTION

HIV continues to be a major global public health issue. In the early years, HIV was unknown, feared, untreatable and often fatal. However, over the past three decades we have come a long way in our understanding of HIV, where it came from, how it evolved, and most importantly, how to treat and prevent it.

HIV attacks and destroys the infection-fighting CD4 cells of the immune system. Loss of CD4 cells makes it hard for the body to fight off infections. HIV medicines prevent HIV from multiplying (making copies of itself), which reduces the amount of HIV in the body. Having less HIV in the body gives the immune system a chance to recover.

Currently available anti-retroviral drugs include Nucleoside Reverse Transcriptase Inhibitors (NRTIs), Non-nucleoside Reverse Transcriptase Inhibitors (NNRTIs), Protease Inhibitors (PIs), Integrase inhibitors or integrase strand transfer inhibitors (INSTIs), Fusion Inhibitors, Entry Inhibitors - CCR5 co-receptor antagonist, HIV integrase strand transfer inhibitors and the like thereof.

Antiretroviral therapy (ART) is the use of HIV medicines to treat HIV infection. ART involves taking a combination of HIV medicines (called an HIV regimen) every day. ART is recommended for everyone with HIV. ART can't cure HIV, but it helps people with HIV live longer, healthier lives.

Combinations of antiretrovirals create multiple obstacles to HIV replication to keep the number of offspring low and reduce the possibility of a superior mutation. If a mutation that conveys resistance to one of the drugs being taken arises, the other drugs continue to suppress reproduction of that mutation. Combination therapies greatly increase the ease with which they can be taken, which in turn increases the consistency with which medication is taken and thus their effectiveness over the long-term. Because of the complexity of selecting and following a regimen and the potential for side effects there lies an importance of taking medications regularly to prevent viral resistance.

Commonly used NRTI drugs have numerous toxicities partly due to the fact that they are analogs of naturally occurring nucleotides and interfere with the activity of numerous cellular functions. Most current Highly Active Anti-retroviral therapy (HAART) regimens include combination of three or more anti-retroviral agents, in common consists of three drugs namely, 2 NRTIs a PI/ NNRTI/ INSTI (e.g: combination of 2 NRTIs (Abacavir + Lamivudine) and 1 INSTI (dolutegravir) is an approved dosage form).

The need to keep plasma levels above a minimum level and the pharmacokinetic properties of the HIV drugs, a frequent administration of relatively high doses is needed. The number of HIV drugs and the amount used in the dosage form pose a common problem referred to as `pill burden'. A high pill burden is undesirable resulting in patient incompliance due to which most of the patients do not take the entire dose and thus fail to comply with the prescribed dosage regimen. The problems associated with high pill burden are multiplied when a combination of various anti-retroviral agents are administered in combination with booster antiretrovirals (e.g: cobicistat) to improve pharmacokinetics.

One limitation of most of the NRTI-sparing regimens has been the above discussed higher pill burden than more other standard regimens. However, the approvals of dolutegravir and the co-formulated darunavir/ cobicistat, both with well-established antiviral activities, may allow for an effective NRTI-sparing regimen. A shift to darunavir, dolutegravir and cobicistat combination in virologically suppressed HIV-infected individuals has the potential to avoid NRTI-associated toxicity while maintaining virologic suppression.

Still there exists a need for the novel combinations of anti-retroviral drugs. Accordingly, inventors of the present invention had developed compositions comprising a protease inhibitor (darunavir), an integrase inhibitor (dolutegravir) and a CYPA3 inhibitor (cobicistat) that has a relatively small size contributing to the convenience of intake and thus help to overcome the problem associated high pill burden and also the multi-class combinations aid to combat the problems associated viral resistance.

Darunavir ethanolate is a protease inhibitor, described chemically as [(1S,2R)-3-[[(4-aminophenyl)sulfonyl](2-methylpropyl)amino]-2-hydroxy-1(phenylmethyl)propyl]-carbamic acid (3R,3aS,6aR)-hexahydrofuro[2,3-b]furan-3-yl ester monoethanolate.

Darunavir ethanolate is commercially available in US as 75mg, 150mg, 600mg & 800mg equivalent to base tablets and as equivalent to 100mg base/ml oral suspension under the brand name PREZISTA^{®}.

Cobicistat is a CYP3A inhibitor, described chemically as 1,3-thiazol-5-ylmethyl [(2R,5R)-5-{[(2S)-2[(methyl{[2-(propan-2-yl)-1,3-thiazol-4-yl]methyl}carbamoyl)amino]-4-(morpholin-4yl)butanoyl]amino}-1,6-diphenylhexan-2-yl].

Cobicistat is commercially available in US as 150mg tablets under the brand name TYBOST^{®}.

Combination of darunavir ethanolate and cobicistat is commercially available in the US market as 800mg equivalent base/ 150 mg under the brand name PREZCOBIX^{®}.

US Patent No. 5,843,946 & 7,700,645 discloses darunavir and its solvates.

PCT Publication No. 2009/013356 assigned to Tibotec disclose tablet compositions comprising darunavir.

US Patent No. 8,148,374 assigned to Gilead discloses cobicistat substance.

PCT Publication No. 2009/135179 assigned to Gilead discloses composition comprising cobicistat and process for preparing the same.

PCT Publication No. 2013/004818 assigned to Janssen discloses compositions comprising darunavir and cobicistat.

US 2016/067255A1 by Gilead Sciences, Inc, relates to methods of treatment and prevention of HIV by administration of the combination of tenofovir alafenamide with dolutegravir. It mentions that this combination can further be combined with one, two or three additional therapeutic agents, selected from a list including practically any therapeutic agent known for HIV treatment.

US 2014/142070 A1 discloses a composition comprising darunavir and cobicistat. It discloses compositions containing a high dose of darunavir (800 mg) and 150 mg of cobicistat for improving compliance.

Dolutegravir sodium is an integrase inhibitor, described chemically as (4R,12aS)-9-{[(2,4-difluorophenyl) methyl]carbamoyl}-4-methyl-6,8-dioxo-3,4,6,8,12,12a-hexahydro-2H-pyrido[1',2':4,5]pyrazino [2,1-b][1,3]oxazin-7-olate sodium.

Dolutegravir sodium is commercially available in US as 10mg, 25mg & 50mg equivalent to base tablets under the brand name TIVICAY^{®}.

US Patent No. 8,129,385 assigned to VIIV Healthcare discloses dolutegravir substance.

### SUMMARY OF THE INVENTION

The present invention relates to pharmaceutical anti-retroviral composition. In particular, the present invention relates to solid oral compositions, comprising combination of multi-class drugs particularly, darunavir, dolutegravir and cobicistat and a process for preparing the same.

The invention is defined by the claims. Any subject-matter falling outside the scope of the claims is provided for information purposes only.

One aspect of the present invention is related to a pharmaceutical composition comprising darunavir, dolutegravir and cobicistat with one or more pharmaceutically acceptable excipients wherein the composition is in the form of a monolithic tablet, comprising a) an intragranular portion comprising combination of darunavir and dolutegravir and one or more pharmaceutically acceptable excipients and b) an extragranular portion comprising of cobicistat and one or more pharmaceutically acceptable excipients, and wherein the total tablet weight ranges from 1600 mg to 1750 mg.

Another aspect of the present invention is related to a wet granulation process for the preparation of a pharmaceutical tablet according to the first aspect of the invention comprising the following steps (a) granules of darunavir are prepared by wet granulation (b) granules of dolutegravir are prepared by wet granulation (c) granules of step (a) and step (b) are mixed for a specified time (d) mixture of granules obtained in step (c) are mixed with extragranular cobicistat and one or more pharmaceutically acceptable excipients (e) blend of step (d) is compressed into tablets and (e) finally, tablets obtained in step (d) are film coated.

A further aspect of the present invention is the pharmaceutical composition according to the first aspect of the invention for use in the treatment of HIV infection in a patient in need thereof.

### DETAILED DESCRIPTION OF THE INVENTION

The present invention relates to solid oral composition comprising combination of multi-class products particularly, darunavir, dolutegravir and cobicistat and process of manufacturing the same.

The term "active agent" as used herein according to the present invention refers to "darunavir", "dolutegravir" and "cobicistat".

The term "darunavir" as used herein according to the present invention includes darunavir in the form of free base or a pharmaceutically acceptable salt, solvate or ester thereof. Preferably, darunavir ethanolate.

The term "dolutegravir" as used herein according to the present invention includes dolutegravir in the form of free base or a pharmaceutically acceptable salt or solvate thereof. Preferably, dolutegravir sodium.

The term "cobicistat" as used herein according to the present invention includes cobicistat in the form of free base or a pharmaceutically acceptable salt thereof. Preferably, cobicistat base.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure and so forth.

The term "excipient" means a pharmacologically inactive component such as a diluent, a binder, a disintegrant, a glidant, a lubricant, etc of a pharmaceutical product. The excipients that are useful in preparing a pharmaceutical composition are generally safe, non-toxic and are acceptable for veterinary as well as human pharmaceutical use. Reference to an excipient includes both one and more than one such excipient.

The term "composition" or "pharmaceutical composition" or "solid oral composition" or "dosage form" as used herein synonymously include solid dosage forms such as tablets, capsules, granules, pellets and the like meant for oral administration.

Pharmaceutical compositions according to the present invention are in the form of monolithic tablets.

The term "monolithic" as used anywhere in the present invention means a single layered tablet composition comprising of darunavir, dolutegravir, cobicistat and one or more pharmaceutically acceptable excipients.

The term "multi-class" as used anywhere in the present invention means anti-retroviral drugs belonging to various categories described in terms of pathway or mechanism of action of the drug. Preferably, multi-class compounds according to the present invention belong to protease inhibitors (e.g: Darunavir), integrase inhibitors (e.g: Dolutegravir) and CYPA3 inhibitors (e.g; Cobicistat).

The first aspect of the present invention is related to a pharmaceutical composition comprising darunavir, dolutegravir and cobicistat with one or more pharmaceutically acceptable excipients, wherein the composition is in the form of a monolithic tablet, comprising a) an intragranular portion comprising combination of darunavir and dolutegravir and one or more pharmaceutically acceptable excipients and b) an extragranular portion comprising of cobicistat and one or more pharmaceutically acceptable excipients, and wherein the total tablet weight ranges from 1600 mg to 1750 mg.

Compositions according to the present invention are prepared either by direct compression or by granulation techniques namely wet granulation and dry granulation. Preferably, the compositions of the invention are prepared by wet granulation.

Suitable granulating medium to prepare granules by wet granulation according to the present invention include binder solution or suspension comprising binder and a solvent. Alternatively, granulation can be carried by using solvent alone.

Solvents include but are not limited to purified water, tertiary-butyl alcohol, isopropyl alcohol, dichloromethane, methanol, methylene chloride and the like and combinations thereof.

In one embodiment, the tablets of the present invention are manufactured by a process comprising steps of:
a) Mannitol, sodium starch glycolate and povidone are sifted through mesh #30,
b) dolutegravir sodium and materials of step (a) are sifted through mesh #30,
c) darunavir ethanolate and materials of step (b) are sifted through mesh #30,
d) blend of step (c) is granulated using purified water to get the desired granules,
e) obtained granules in step (d) are dried at 60°C temperature and sifted through mesh #20,
f) silicon dioxide adsorbed cobicistat is sifted through mesh #40,
g) silicified microcrystalline cellulose, colloidal silicon dioxide and crospovidone together are sifted through mesh #40,
h) materials of step (f) and (g) are blended together for 10 mins,
i) sodium stearyl fumarate is sifted through mesh #40,
j) blend of step (h) is lubricated with sifted sodium stearyl fumarate of step (i),
k) lubricated blend of step (j) is compressed into tablets,
l) finally, tablets obtained in step (k) are film coated.

In another embodiment, the pharmaceutical tablet compositions of the present invention are prepared by a process comprising steps of:
*Part 1* - *Darunavir Granulation:*
   a) Darunavir is sifted through a mesh #40,
   b) granulating medium is prepared by dissolving hypromellose in purified water,
   c) sifted darunavir of step (a) is granulated using the medium prepared in step (b)
   d) granules obtained in step (c) are dried at 50°C temperature,
   e) dried granules of step (d) are sifted through mesh# 30 to obtain desired size granules.
*Part 2* - *Dolutegravir granulation:*
   a) Dolutegravir, mannitol and sodium starch glycolate are sifted through mesh#30,
   b) microcrystalline cellulose and povidone are sifted through mesh #40
   c) materials of step (a) and (b) are sifted together through mesh#30 and mixed for 10 mins,
   d) blend of step (c) is granulated using purified water
   e) granules obtained in step (d) are dried at 60°C temperature,
   f) dried granules of step (e) are sifted through mesh #35 to obtain desired size granules.
*Part 3* - *Extra-granular sifting:*
   a) silicon dioxide adsorbed cobicistat is sifted through mesh #40
   b) silicified microcrystalline cellulose, colloidal silicon dioxide and crospovidone are co-sifted through mesh #40,
   c) materials of step (a) and (b) are blended together for 10 mins,
   d) dry mix the granules obtained in Part 1 and Part 2 for 10 mins,
   e) pre-lubricate the mixed granules of step (d) with the blend of step (c)
   f) sodium stearyl fumarate is sifted through mesh #40
   g) pre-lubricated blend of step (e) is lubricated finally with sifted sodium stearyl fumarate of step (f),
   h) compress the lubricated blend of step (g) into tablets, and
   i) finally, tablets obtained in step (h) are film coated.

One another embodiment of the present invention is related to pharmaceutical unitary tablet composition comprising a) 800 mg of darunavir or its pharmaceutically acceptable salt thereof, b) 50 mg of dolutegravir or its pharmaceutically acceptable salt thereof, c) 150 mg of cobicistat and d) one or more pharmaceutically acceptable excipients, wherein the total tablet weight ranges from 1600 mg to 1750 mg.

The pharmaceutical tablet composition according to the present invention comprise excipients selected from diluents, binders, disintegrants, lubricants, glidants and combinations thereof.

Diluents include but are not limited to starches, modified starches, lactose, dibasic calcium phosphate, tribasic calcium phosphate, microcrystalline cellulose, silicified microcrystalline cellulose, mannitol, calcium carbonate, calcium sulfate, talc, sugar, magnesium carbonate, magnesium oxide and the like or combinations thereof.

Binders include but are not limited to hydroxypropyl cellulose, low-substituted hydroxypropyl cellulose, hydroxypropyl methylcellulose or hypromellose, polyvinyl pyrrolidone (povidone), pregelatinized starch, powdered acacia, gelatin, guar gum, carbomers and the like or combinations thereof.

Disintegrants include but are not limited to croscarmellose sodium, sodium starch glycolate, crospovidone, polacrillin potassium, microcrystalline cellulose, polyvinylpyrrolidone, carboxymethyl cellulose calcium, starches such as corn starch, potato starch and modified starches, clays, bentonite, microcrystalline cellulose and the like or combinations thereof.

Lubricants include but are not limited to talc, magnesium stearate, calcium stearate, zinc stearate, stearic acid, palmitic acid, sodium stearyl fumarate, carnauba wax, hydrogenated vegetable oils, mineral oil, polyethylene glycols, and the like or combinations thereof.

Glidants include but are not limited to colloidal silicon dioxide, other forms of silicon dioxide, such as aggregated silicates, hydrated silica, magnesium silicate, magnesium trisilicate, talc, and the like or combinations thereof.

Advantageously, the tablet compositions of the present invention comprising three active ingredients particularly darunavir, dolutegravir and cobicistat, still can be formulated in an acceptable size that account for a reduced total tablet weight ranging from 1600 mg to 1750 mg. The said size and weight of the tablet dosage form thus can overcome the effect of pill burden.The solid oral dosage forms of the present invention are film coated with an aqueous or non-aqueous solution comprising film forming polymers and one or more of plasticizers, opacifiers, anti-tacking agents, coloring agents and the like and combinations thereof.

A film coat on the tablet provides an elegant appearance, protects from moisture and further contributes to the ease with which it can be swallowed.

Film coating composition according to the present invention is polymer based. Suitable polymers include alkyl celluloses such as methyl cellulose, ethyl cellulose, hydroxypropyl cellulose, hydroxypropyl methyl cellulose, cellulose acetate, poly vinyl alcohol, polyvinyl acetate, poly vinyl chloride, polyvinyl pyrrolidone and the like and combinations thereof.

Plasticizers include but are not limited to glyceryl monostearate, triethyl citrate, macrogols, lactic acid, lactic acid acetamide, sorbitol, glycerin, triacetin, acetyl triethyl citrate, acetyl tributyl citrate, polyvinylpyrrolidone, triethylene glycol, ethylene glycol monooleate, acetylated monoglycerides, cetyl alcohol and other hydrogenated oils and waxes, as well as polyethylene glycol and the like or combinations thereof.

Yet another embodiment of the present invention is related to process of preparing a pharmaceutical tablet comprising the step of (a) granules of darunavir were prepared by wet granulation (b) granules of dolutegravir were prepared by wet granulation (c) granules of step (a) and step (b) were mixed for a specified period of time (d) mixture of granules obtained in step (c) were mixed with extragranular cobicistat and one or more pharmaceutically acceptable excipients (e) blend of step (d) was compressed into tablets and (e) finally, tablets obtained in step (d) were film coated.

The present invention also provides the pharmaceutical composition according to the invention for use in the treatment of HIV infection in a patient in need thereof.Certain specific aspects and embodiments of this invention are described in further detail by the examples below, which are provided only for the purpose of illustration and are not intended to limit the scope of the invention in any manner.

### EXAMPLES

### Example 1

### Single layer tablet Composition:

| **Ingredient** | **mg/tab** |
|---|---|
| **Intragranular Portion** | |
| *Darunavir Granulation* | |
| Darunavir ethanolate | 867.28 |
| Hypromellose | 13.20 |
| Purified water | q.s |

| *Dolutegravir Granulation* | |
|---|---|
| Dolutegravir sodium | 52.60 |
| Microcrystalline cellulose | 59.00 |
| Mannitol | 140.40 |
| Sodium starch glycolate | 15.00 |
| Povidone | 15.00 |
| Purified water | q.s |

| **Extragranular Portion** | |
|---|---|
| Cobicistat on silicon dioxide | 288.00 |
| Silicified Microcrystalline cellulose | 164.52 |
| Colloidal silicon dioxide | 17.00 |
| Crospovidone | 51.00 |
| Sodium stearyl fumarate | 17.00 |
| **Core tablet weight** | **1700.00** |
| **Film coating with Opadry** | |
| **Coated tablet weight** | **1742.50** |

### Brief manufacturing process:

*Part 1* - *Darunavir Granulation:*
   a) Darunavir was sifted through a mesh #40,
   b) granulation medium was prepared by dissolving hypromellose in purified water,
   c) sifted darunavir of step (a) was granulated using the medium prepared in step (b)
   d) granules obtained in step (c) were dried at 50°C temperature,
   e) dried granules of step (d) were sifted through mesh# 30 to obtain desired size granules.
*Part 2* - *Dolutegravir granulation:*
   a) Dolutegravir, mannitol and sodium starch glycolate were sifted through mesh#30,
   b) silicified microcrystalline cellulose and povidone were sifted through mesh #40
   c) materials of step (a) and (b) were sifted together through mesh#30 and mixed for 10 mins,
   d) blend of step (c) was granulated using purified water
   e) granules obtained in step (d) were dried at 60°C temperature,
   f) dried granules of step (e) were sifted through mesh #35 to obtain desired size granules.
*Part 3* - *Extra-granular sifting:*
   a) silicon dioxide adsorbed cobicistat was sifted through mesh #40
   b) silicified microcrystalline cellulose, colloidal silicon dioxide and crospovidone were co-sifted through mesh #40,
   c) materials of step (a) and (b) were blended together for 10 mins,
   d) dry mix the granules obtained in Part 1 and Part 2 for 10 mins,
   e) pre-lubricate the mixed granules of step (d) with the blend of step (c)
   f) sodium stearyl fumarate was sifted through mesh #40
   g) pre-lubricated blend of step (e) was lubricated finally with sifted sodium stearyl fumarate of step (f),
   h) compress the lubricated blend of step (g) into tablets, and
   i) finally, tablets obtained in step (h) were film coated.

### Comparative dissolution data:

### Dissolution test conditions:

| | |
|---|---|
| Apparatus : | USP apparatus II (Paddle type) |
| rpm : | 100 rpm |
| pH : | 3.0 |
| Media : | 0.05M sodium phosphate buffer containing 2% Tween 20 |
| Volume: : | 900ml |

**Table 1: Comparative dissolution profile:**

| Time in minutes | **% drug released** | | | | | |
|---|---|---|---|---|---|---|
| | **Example 1 (Single layer tablet)** | | | **Innovator plain marketed tablets** | | |
| | Darunavir | Cobicistat | Dolutegravir | Darunavir (Prezista^{®}) | Cobicistat (Tybost^{®}) | Dolutegravir (Tivicay^{®}) |
| 10 | 51 | 91 | 83 | 41 | 86 | 76 |
| 15 | 64 | 93 | 90 | 56 | 87 | 84 |
| 20 | 71 | 94 | 94 | 63 | 87 | 88 |
| 30 | 81 | 95 | 96 | 74 | 88 | 90 |
| 45 | 88 | 95 | 98 | 82 | 89 | 93 |
| Infinity | 92 | 96 | 101 | 86 | 91 | 96 |

### Example 2

### Single layer tablet Composition:

| **Ingredient** | **mg/tab** |
|---|---|
| **Intra-granular Portion** | |
| Darunavir ethanolate | 867.28 |
| Dolutegravir sodium | 52.60 |
| Mannitol | 54.98 |
| Sodium starch glycolate | 32.00 |
| Povidone (K-30) | 42.50 |
| Purified water | q.s |

| **Extragranular Portion** | |
|---|---|
| Cobicistat on silicon dioxide | 288.00 |
| Silicified microcrystalline cellulose | 177.64 |
| Colloidal silicon dioxide | 17.00 |
| Crospovidone | 51.00 |
| Sodium stearyl fumarate | 17.00 |
| **Core tablet weight** | **1600.00** |
| **Film coating with Opadry** | |
| **Coated tablet weight** | **1642.50** |

### Brief manufacturing process:

a) Mannitol, sodium starch glycolate and povidone were sifted through mesh #30,
b) dolutegravir sodium and materials of step (a) were sifted through mesh #30,
c) darunavir ethanolate and materials of step (b) were sifted through mesh #30,
d) blend of step (c) was granulated using purified water to get the desired granules,
e) obtained granules in step (d) were dried at 60°C temperature and sifted through mesh #20,
f) silicon dioxide adsorbed cobicistat was sifted through mesh #40,
g) silicified microcrystalline cellulose, colloidal silicon dioxide and crospovidone together were sifted through mesh #40,
h) materials of step (f) and (g) were blended together for 10 mins,
i) sodium stearyl fumarate was sifted through mesh #40,
j) blend of step (h) was lubricated with sifted sodium stearyl fumarate of step (i),
k) lubricated blend of step (j) was compressed into tablets,
l) finally, tablets obtained in step (k) were film coated.

## Claims

1. A pharmaceutical composition comprising darunavir, dolutegravir and cobicistat with one or more pharmaceutically acceptable excipients,
wherein the composition is in the form of a monolithic tablet, wherein
a) an intragranular portion comprising combination of darunavir and dolutegravir and one or more pharmaceutically acceptable excipients and
b) an extragranular portion comprising of cobicistat and one or more pharmaceutically acceptable excipients,
and wherein the total tablet weight ranges from 1600 mg to 1750 mg.

2. A pharmaceutical unitary tablet composition according to claim 1, comprising:
a) 800 mg of darunavir or its pharmaceutically acceptable salt thereof,
b) 50 mg of dolutegravir or its pharmaceutically acceptable salt thereof,
c) 150 mg of cobicistat and
d) one or more pharmaceutically acceptable excipients.

3. A pharmaceutical composition according to claim 1, wherein one or more excipients are selected from diluents, binders, disintegrants, lubricants, glidants and combinations thereof.

4. A pharmaceutical composition according to claim 1, wherein darunavir is in the form of darunavir ethanolate and dolutegravir is in the form of dolutegravir sodium.

5. A pharmaceutical composition according to claim 2 comprising the following:
| **Ingredient** |
|---|
| **Intragranular Portion** |
| *Darunavir Granulation* |
| Darunavir Ethanolate |
| Hypromellose |
| Purified water |
| *Dolutegravir Granulation* |
|---|
| Dolutegravir Sodium |
| MCC |
| Mannitol |
| Sodium starch glycolate |
| Povidone |
| Purified water |
| **Extragranular Portion** |
|---|
| Cobicistat on silicon dioxide |
| Silicified MCC |
| Colloidal silicon dioxide |
| Crospovidone |
| Sodium stearyl fumarate |

6. A process of preparing a pharmaceutical tablet composition according to claim 1, using wet granulation comprising the steps of:
a) Darunavir is sifted through a suitable mesh,
b) granulation medium is prepared by dissolving hypromellose in purified water,
c) sifted darunavir of step (a) is granulated using the medium prepared in step (b) and obtained granules are dried and sifted to obtain uniform size,
d) dolutegravir and one or more additional excipients are sifted separately,
e) materials of step (d) are granulated using purified water and obtained granules are dried and sifted to obtain uniform size,
f) granules of step (c) and step (e) are mixed for a specified time,
g) extragranular cobicistat along with one or more pharmaceutically acceptable excipients are sifted and blended,
h) mixture of granules obtained in step (f) are mixed with the blend of step (g),
i) blend of step (h) is compressed into tablets,
j) finally, tablets obtained in step (i) are film coated.

7. A pharmaceutical composition according to claim 1 for use in the treatment of HIV infection in a patient in need thereof.

## Patentansprüche

1. Eine pharmazeutische Zusammensetzung umfassend Darunavir, Dolutegravir und Cobicistat mit einem oder mehreren pharmazeutisch akzeptablen Hilfsstoffen,
wobei die Zusammensetzung in Form einer monolithischen Tablette vorliegt, wobei
a) einen intragranulären Anteil umfassend eine Kombination aus Darunavir und Dolutegravir und einem oder mehreren pharmazeutisch akzeptablen Hilfsstoffen, und
b) einen extragranulären Anteil umfassend Cobicistat und einen oder mehrere pharmazeutisch akzeptable Hilfsstoffe,
und wobei das Gesamtgewicht der Tablette im Bereich von 1600 mg bis 1750 mg liegt.

2. Eine pharmazeutische Einheitstablettenzusammensetzung nach Anspruch 1, umfassend:
a) 800 mg Darunavir oder sein pharmazeutisch akzeptables Salz davon,
b) 50 mg Dolutegravir oder sein pharmazeutisch akzeptables Salz davon,
c) 150 mg Cobicistat und
d) einen oder mehrere pharmazeutisch akzeptable Hilfsstoffe.

3. Eine pharmazeutische Zusammensetzung nach Anspruch 1, wobei ein oder mehrere Hilfsstoffe ausgewählt sind aus Verdünnungsmitteln, Bindemitteln, Zerfallsbeschleunigern, Gleitmitteln, Fließregulierungsmitteln und Kombinationen davon.

4. Eine pharmazeutische Zusammensetzung nach Anspruch 1, wobei das Darunavir in Form von Darunavir-Ethanolat und das Dolutegravir in Form von Dolutegravir-Natrium vorliegt.

5. Eine pharmazeutische Zusammensetzung nach Anspruch 2, umfassend das Folgende:
| **Zutat** |
|---|
| **Intragranulärer Anteil** |
| *Darunavir Granulierung* |
| Darunavir-Ethanolat |
| Hypromellose |
| Gereinigtes Wasser |
| *Dolutegravir Granulierung* |
|---|
| Dolutegravir-Natrium |
| MCC |
| Mannitol |
| Natriumstärkeglykolat |
| Povidon |
| Gereinigtes Wasser |
| **Extragranulärer Anteil** |
|---|
| Cobicistat auf Siliciumdioxid |
| Silizierte MCC |
| Kolloidales Siliciumdioxid |
| Crospovidon |
| Natriumstearylfumarat |

6. Ein Verfahren zur Herstellung einer pharmazeutischen Tablettenzusammensetzung nach Anspruch 1 unter Verwendung von Nassgranulation, umfassend die folgenden Schritte:
a) Darunavir wird durch ein geeignetes Sieb gesiebt,
b) ein Granulierungsmedium wird durch Auflösen von Hypromellose in gereinigtem Wasser hergestellt,
c) das gesiebte Darunavir von Schritt (a) wird unter Verwendung des in Schritt (b) hergestellten Mediums granuliert und das erhaltene Granulat wird getrocknet und gesiebt, um eine einheitliche Größe zu erhalten,
d) das Dolutegravir und ein oder mehrere zusätzliche Hilfsstoffe werden getrennt gesiebt,
e) die Materialien von Schritt (d) werden unter Verwendung von gereinigtem Wasser granuliert und das erhaltene Granulat wird getrocknet und gesiebt, um eine einheitliche Größe zu erhalten;
f) das Granulat von Schritt (c) und Schritt (e) wird für eine bestimmte Zeit gemischt,
g) das extragranulare Cobicistat zusammen mit einem oder mehreren pharmazeutisch akzeptablen Hilfsstoffen werden gesiebt und gemischt,
h) das Granulatgemisch, das in Schritt (f) erhalten wurde, wird mit der Mischung aus Schritt (g) gemischt,
i) die Mischung aus Schritt (h) wird zu Tabletten verpresst,
j) schließlich werden die in Schritt (i) erhaltenen Tabletten filmbeschichtet.

7. Eine pharmazeutische Zusammensetzung nach Anspruch 1 zur Verwendung bei der Behandlung einer HIV-Infektion bei einem Patienten, der diese benötigt.

## Revendications

1. Une composition pharmaceutique comprenant du darunavir, du dolutégravir et du cobicistat avec un ou plusieurs excipients pharmaceutiquement acceptables,
dans laquelle la composition est sous la forme d'un comprimé monolithique, dans lequel
a) une partie intragranulaire comprenant une combinaison de darunavir et de dolutégravir et un ou plusieurs excipients pharmaceutiquement acceptables et
b) une partie extragranulaire comprenant du cobicistat et un ou plusieurs excipients pharmaceutiquement acceptables,
et dans laquelle le poids total du comprimé va de 1600 mg à 1750 mg.

2. Une composition de comprimé unitaire pharmaceutique selon la revendication 1, comprenant :
a) 800 mg de darunavir ou son sel pharmaceutiquement acceptable de celui-ci,
b) 50 mg de dolutégravir ou son sel pharmaceutiquement acceptable de celui-ci,
c) 150 mg de cobicistat et
d) un ou plusieurs excipients pharmaceutiquement acceptables.

3. Une composition pharmaceutique selon la revendication 1, dans laquelle un ou plusieurs excipients sont choisis parmi des diluants, des liants, des agents de désagrégation, des agents lubrifiants, des agents de glissement et des combinaisons de ceux-ci.

4. Une composition pharmaceutique selon la revendication 1, dans laquelle le darunavir est sous la forme d'éthanolate de darunavir et le dolutégravir est sous la forme de dolutégravir sodique.

5. Une composition pharmaceutique selon la revendication 2, comprenant les composantes suivantes :
| **Ingrédient** |
|---|
| **Partie Intragranulaire** |
| *Granulation de Darunavir* |
| Éthanolate de darunavir |
| Hypromellose |
| Eau purifiée |
| *Granulation de Dolutégravir* |
|---|
| Dolutégravir sodique |
| MCC |
| Mannitol |
| Glycolate d'amidon sodique |
| Povidone |
| Eau purifiée |
| **Partie Extragranulaire** |
|---|
| Cobicistat sur dioxyde de silicium |
| MCC silicatée |
| Dioxyde de silicium colloïdal |
| Crospovidone |
| Stéaryl fumarate de sodium |

6. Un procédé de préparation d'une composition pharmaceutique en comprimés selon la revendication 1, en utilisant une granulation par voie humide, comprenant les étapes consistant à :
a) le darunavir est tamisé à travers un tamis approprié,
b) un milieu de granulation est préparé par dissolution de l'hypromellose dans de l'eau purifiée,
c) le darunavir tamisé de l'étape (a) est granulé à l'aide du milieu préparé à l'étape (b) et les granulés obtenus sont séchés et tamisés pour obtenir une taille uniforme,
d) le dolutégravir et un ou plusieurs excipients supplémentaires sont tamisés séparément,
e) les matériaux de l'étape (d) sont granulés à l'aide d'eau purifiée et les granulés obtenus sont séchés et tamisés pour obtenir une taille uniforme,
f) les granulés de l'étape (c) et de l'étape (e) sont mélangés pendant un temps déterminé,
g) le cobicistat extragranulaire avec un ou plusieurs excipients pharmaceutiquement acceptables sont tamisés et mélangés,
h) le mélange de granulés obtenu à l'étape (f) sont mêlés avec le mélange de l'étape (g),
i) le mélange de l'étape (h) est comprimé en obtenant des comprimés,
j) enfin, les comprimés obtenus à l'étape (i) sont pelliculés.

7. Une composition pharmaceutique selon la revendication 1, pour l'utilisation dans le traitement d'une infection par le VIH chez un patient qui en a besoin.
